# EUROPEAN PATENT APPLICATION

(11) **EP 4 756 721 A1**
(43) Date of publication of application: **10.06.2026**
(21) Application number: 25214612.1
(22) Date of filing: 10.11.2025
(51) Int. Cl.: G06T 12/30

(54) **MAMMOGRAPHY IMAGING SYSTEM WITH ENHANCED IMAGE RESOLUTON AND METHOD OF USE**

(30) Priority: 05.12.2024 US 202418969928
(71) Applicant: GE Precision Healthcare LLC, Waukesha, WI 53188 (US)
(72) Inventor: QUILLENT, Arnaud, 75014 Paris (FR); BISMUTH, Vincent, 92190 Meudon (FR); KLAUSZ, Rémy, 92200 Neuilly-sur-Seine (FR); MILIONI DE CARVALHO, Pablo, 92370 Chaville (FR)
(74) Representative: Kilburn & Strode LLP

(57) **Abstract**

An imaging device or system (10), e.g., a mammography imaging system (12), and associated method provides artefact-reduced images (420,422,424) of an object (52). The system (10) and method generates a standard reconstructed volume (410) of the object (52) created from one or more projection images (404) obtained of the object within an angular range of less than +/- 60 degrees relative to the object (52). A resolution enhancement artificial intelligence (414) is applied to the projection images (404) to form an artefact-reduced/quasi-isotropic volume (416), and one or more quasi-isotropic images (420,422,424) obtained therefrom along planes that are orthogonal to the plane of the detector (18) for the mammography imaging system (12) . The resolution of the quasi-isotropic images (420,422,424) is similar to images obtained in a simulated computed tomography imaging procedure and the quasi-isotropic volume (416) can be utilized to provide effective diagnostic images using strain elastography and the digital decompression of the quasi-isotropic volume (416) to create a modifiable digital uncompressed object volume (800).

## Description

### FIELD OF THE DISCLOSURE

The present disclosure relates generally to medical imaging systems, including mammography systems and devices, and more specifically to the generation and utilization of enhanced resolution images produced from mammography systems.

### BACKGROUND OF THE DISCLOSURE

Embodiments of the invention relate generally to X-ray medical imaging, and more particularly to devices, systems and methods employed to perform various imaging procedures, such as mammography imaging procedures including but not limited to digital breast tomosynthesis (DBT) mammography exams, spectral mammography (SM), such as 2D/3D dual-energy contrast-enhanced (CE) mammography exams or full-field digital mammography (FFDM) .

For breast imaging, some exemplary imaging processes include full-field digital mammography, which captures the image directly onto a flat-panel detector, computed radiography, which involves the use of a cassette that contains an imaging plate. Additionally, spectral mammography (SM) can be employed which is an X-ray imaging modality used to scan breasts for screening, diagnosis and/or interventional examinations. However, the effectiveness of these types of mammography imaging is affected by numerous factors, one of which is the two-dimensional (2D) rendering of images obtained using them.

As an improvement to the above mammography imaging processes that produce only 2D images, of the breast digital breast tomosynthesis (DBT) system is a dedicated mammography system that acquires several (e.g., tens of and/or between 9-40) angularly offset projection X-ray images over a limited angular range relative to the breast. The DBT system can use the resulting X-ray image data to reconstruct three-dimensional (3D) image datasets that show the full volume of the breast with differentiation in the direction orthogonal to the detector plane (the "z-direction").

The 3D image datasets are used to form various volumetric representations of the imaged breast, including an entire 3D volume of the breast, and various 3D sections of the 3D volume, such as tomographic planes or slices of predefined thicknesses of the 3D volume oriented to provide the desired view of one or more regions of interest (ROI) detected within the 3D image dataset. However, these tomographic planes results from the limited angle over which the projections are acquired and include undesired information from regions above and below the generated tomographic plane with no hard limit, resulting in undefined or unclear borders of the tomographic planes from the 3D image dataset. Due to the limited angular range of tomosynthesis acquisitions the volumes are presented in a very anisotropic representation where the pixel spacing/spatial resolution in the axial plane is the same order of magnitude as in the detector, and an order of magnitude less in the orthogonal direction.

In addition, when the 3D image datasets of the breast have been produced, after being utilized in a suitable diagnosis procedure, they can be utilized to guide a biopsy device employed with the DBT system into the breast to obtain a biopsy of the region of interest (ROI) identified within the 3D image datasets. In DBT systems, the biopsy device is disposed directly on the DBT system in order to be able to perform the biopsy utilizing the 3D image dataset or a stereo-pair of camera images of the breast and biopsy device with a subsequent triangulation of the biopsy device to the ROI in the breast to guide the biopsy device to the ROI.

With regard to the use of these DBT mammography systems, the set-up of the system to obtain the images requires the attachment of various devices to the system in order to provide the system with the proper positioning, i.e., compression, of the breast to obtain the image quality desired. In mammography systems, the devices that are attached to the system to perform the imaging and/or biopsy procedure include a compression paddle, a magnification device, and/or a biopsy holder, which is utilized to locate the biopsy device on the mammography system in a location where the biopsy device can perform the desired biopsy procedure under the guidance of the mammography system. When the mammography imaging system is operated in a screening configuration, the compression paddle, and optionally the magnification device (or magstand), are connected to the system. Conversely, when the imaging system is employed in a diagnostic configuration, optionally along with the magnification device, the biopsy positioner or holder and a compression paddle compatible with the operation of the biopsy device on the biopsy holder are secured to the imaging system. In both configurations, the detector is fixed as a part of the imaging system, or can be rotated to follow the angle of the source inside a fixed breast support, with the bucky secured to the detector to provide a suitable x-ray transparent breast support surface along with the image enhancing, anti-scatter grid located within the bucky.

In prior art diagnostic mammography imaging devices, such as a DBT system, the radiation source is positioned directly above the detector, with the object being imaged, e.g., the breast, disposed in a compressed position on adjacent the detector. In this configuration, x-rays emitted by the radiation source pass through the breast along the axis defined between the radiation source and the detector to generate a 2D projection image of the breast. Further, the radiation source can be moved or rotated into difference angular positions relative to the compressed breast and the detector in order to generate the additional 2D projection images employed by the mammography imaging device/DBT system to reconstruct a 3D volume of the breast.

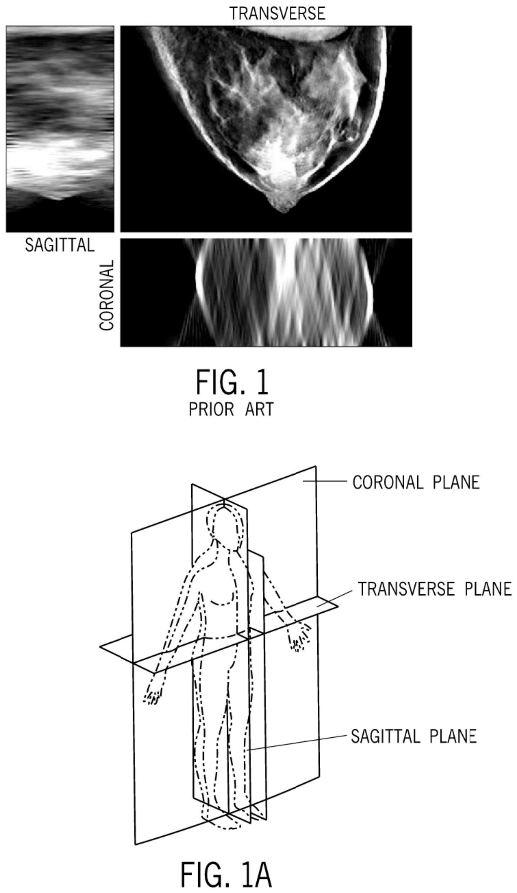

However, due to the geometry of the DBT system, i.e., the limited angular range, strong artefacts appear in the 3D volumes reconstructed from the acquired 2D X-ray projections along certain planes. Most notably, the resolution along vertical detector-to-source axes, e.g., along the coronal plane, the sagittal plane, and/or any other plane orthogonal to the detector or along any line between the detector and the focal point of the radiation source, is severely reduced, and breast structures superimposed in this direction cannot be distinctly separated. An example of the current state of the art for reprojected images obtained from the reconstructed 3D volume along the coronal, sagittal and transverse or axial planes is shown in FIGS. 1 and 1A. As illustrated, only the image along the transverse plane, i.e., the plane perpendicular to the vertical detector-to-source axis, provides useful diagnostic information. Consequently, radiologists cannot currently leverage information coming from all directions of sight of the 3D volume as only one of them is usable due to the geometrical orientation of the artefacts. In particular, it not possible to generate diagnostically acceptable tomographic image planes other than those planes oriented sensibly parallel to the detector, and even those image planes can be "polluted" by the images of the large structures present in adjacent transverse planes above and/or below the tomographic image plane.

Over the past years the development of reconstruction algorithms leveraging AI techniques has enabled to produce reconstructed volumes with improved image quality vertical source-to-detector axes. However, imaging system designs, image review methods and applications leveraging these advances have not been studied yet.

Therefore, with regard to the aforementioned shortcomings of prior art mammography imaging systems concerning the image quality along vertical source-to-detector axes, it is desirable to develop a mammography system and associated method for producing improved reprojected images along vertical source-to-detector planes to provide enhanced image information for use in diagnostic and treatment procedures

### SUMMARY OF THE DISCLOSURE

According to one aspect of an exemplary embodiment of the present disclosure, a mammography imaging system includes a radiation source operable to emit radiation, a detector alignable with the radiation source, the detector having a surface on which a breast to be imaged is adapted to be positioned, a controller operably connected to the radiation source and the detector to control the operation of the radiation source and detector to generate image data of the breast in an imaging procedure performed by the imaging system, the controller including a central processing unit and interconnected database containing processor-executable instructions for processing the image data from the detector to create one or more projection images of the breast, a display operably connected to the controller for presenting information to a user, and a user interface operably connected to the controller to enable user input to the controller, wherein the controller is configured to apply a resolution enhancement artificial intelligence to the one or more projection images to reconstruct a quasi-isotropic volume of the breast, and to generate one more one or more quasi-isotropic images from the quasi-isotropic volume.

According to still another aspect of an exemplary embodiment of the present disclosure, a method for providing one or more projection images obtained of the object within an angular range of less than 180 degrees relative to the object, reconstructing a quasi-isotropic volume from the one or more projection images with an artefact-reduction and resolution enhancement artificial intelligence and generating one or more quasi-isotropic images from the quasi-isotropic volume.

According to still another aspect of an exemplary embodiment of the present disclosure, a method for providing artefact-reduced images of an object includes the steps of providing a mammography imaging system including a radiation source operable at to emit radiation, a detector alignable with the radiation source, the detector having a surface on which the breast to be imaged is adapted to be positioned, a controller operably connected to the radiation source and the detector to control the operation of the radiation source and detector to generate image data of the breast in an imaging procedure performed by the imaging system, the controller including a central processing unit and interconnected database containing processor-executable instructions for processing the image data from the detector to create one or more projection images, a display operably connected to the controller for presenting information to a user; and a user interface operably connected to the controller to enable user input to the controller, placing the breast on the surface of the detector, operating the radiation source over a limited angular range relative to the breast to obtain image data, processing the image data to form the one or more projection images; reconstructing a quasi-isotropic volume from the one or more projection images with an artefact-reduction and resolution enhancement artificial intelligence, and generating one or more quasi-isotropic images from the quasi-isotropic volume..

These and other exemplary aspects, features and advantages of the invention will be made apparent from the following detailed description taken together with the drawing figures.

### BRIEF DESCRIPTION OF THE DRAWINGS

The drawings illustrate the best mode currently contemplated of practicing the present invention.

In the drawings:
FIGS. 1 and 1A are schematic views of various prior art images sampled along various axes of an image volume produced by current state of the art mammography imaging systems and an axis reference for the various images.
FIG. 2 is a perspective view of an imaging device in the form of a mammography system for imaging the breast tissue of a patient, in accordance with an embodiment of the disclosure.
FIG. 3 is a diagram of the system of FIG. 2, showing the radiation source of the mammography system in a scanning position, in accordance with an embodiment of the disclosure.
FIG. 4 is a flowchart of the steps of an exemplary method of operation of the mammography system of FIG. 2 to produce an artefact-reduced 3D volume, in accordance with an embodiment of the disclosure.
FIG. 5 is a schematic view of a display including a transverse plane view obtained from the standard reconstructed volume presented with a coronal plane view and sagittal plane view obtained from the artefact-reduced volume produced by the method of FIG. 4, in accordance with an embodiment of the disclosure.
FIG. 6 are schematic views of various synthetic images along various planes obtained in a simulated computed tomography (CT) imaging procedure of the breast compressed and positioned as in a standard DBT system, imaged to produce the images in FIG. 5 as a reference for the accuracy of the images of FIG. 5, in accordance with an embodiment of the disclosure.
FIG. 7 are schematic views of various images sampled along the transverse, coronal and sagittal planes generated from the artefact-reduced volume produced by the method of FIG. 4, in accordance with an embodiment of the disclosure.
FIGS. 8A and 8B are schematic views of a method of operation of the method of FIG. 4 to output slabs (FIG. 8A) and synthetic 2D images (FIG. 8B) from the artefact-reduced volume, in accordance with an embodiment of the disclosure.
FIG. 9 is a volumetric image derived from an artefact-reduced volume produced by the method of FIG. 4, in accordance with an embodiment of the disclosure.
FIG. 10 is a prior art display provided for planning a biopsy procedure including a transverse plane view obtained from the standard reconstructed volume indicating a biopsy target presented with a schematic view of the breast being biopsied along with a representation of the biopsy needle as positioned for performing the biopsy procedure.
FIG. 11 is a display provided for planning a biopsy procedure including a transverse plane view obtained from the standard reconstructed volume indicating a biopsy target presented with a sagittal plane view of the breast being biopsied obtained from the artefact-reduced volume produced by the method of FIG. 4, along with a representation of the biopsy needle as positioned for performing the biopsy procedure, in accordance with an embodiment of the disclosure.
FIG. 12 shows transverse and coronal plane views of the artefact-reduced volume produced by the method of FIG. 4, along with a representation of an 3D ellipsoid annotation applied to the transverse plane view and coronal plane view, in accordance with an embodiment of the disclosure.
FIG. 13 is a schematic view of a transverse plane view of the breast obtained from the artefact-reduced volume produced by the method of FIG. 4, along with a representation of an uncertainty map and score for the transverse plane view, in accordance with an embodiment of the disclosure.
FIGS. 14A-14D shows various schematic views of simulated compression on an uncompressed breast and simulated de-compression of a compressed breast obtained through mechanical numerical simulation of the artefact-reduced volume produced by the method of FIG. 4, in accordance with an embodiment of the disclosure.
FIGS. 15A-15C illustrate images of the results of a tissue density estimation obtained, from a reconstructed volume (a), an artefact-reduced volume produced by the method of FIG. 4 (b), and a material composition image (c) derived from the artefact reduced volume, in accordance with an embodiment of the disclosure.
FIG. 16 is a schematic view of the mammography system of FIG. 2 employed to perform an x-ray strain elastography imaging procedure, in accordance with an embodiment of the disclosure.
FIG. 17 is a flowchart of the steps of an exemplary method of operation of the mammography system of FIG. 2 to perform the x-ray strain elastography imaging procedure, in accordance with an embodiment of the disclosure.
FIGS. 18A-18B are schematic views illustrating the angular coverage of x-ray acquisitions of a spherical object and the modification produced by the method of FIG. 4 to generate an artefact-reduced volume with quasi-isotropic resolution.

### DETAILED DESCRIPTION OF THE DRAWINGS

One or more specific embodiments will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present invention, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be nonlimiting, and thus additional numerical values, ranges, and percentages are within the scope of the disclosed embodiments.

As used herein, "electrically coupled", "electrically connected", and "electrical communication" mean that the referenced elements are directly or indirectly connected such that an electrical current may flow from one to the other. The connection may include a direct conductive connection, i.e., without an intervening capacitive, inductive or active element, an inductive connection, a capacitive connection, and/or any other suitable electrical connection. Intervening components may be present.

Further, while the embodiments disclosed herein are described with respect to a mammography apparatus for digital breast tomosynthesis (DBT), it is to be understood that embodiments of the invention may be applicable to other types of imaging devices for both 2-dimensional and 3-dimensional imaging including, for example, fluoroscopy, full-field digital mammography, the 2-dimensional imaging of breast tissue and spectral mammography (single or multi-energy), as well as for imaging procedures for tissue other than breast tissue. Further still, embodiments of the invention may be used to analyze tissue, generally, and are not limited to analyzing human tissue.

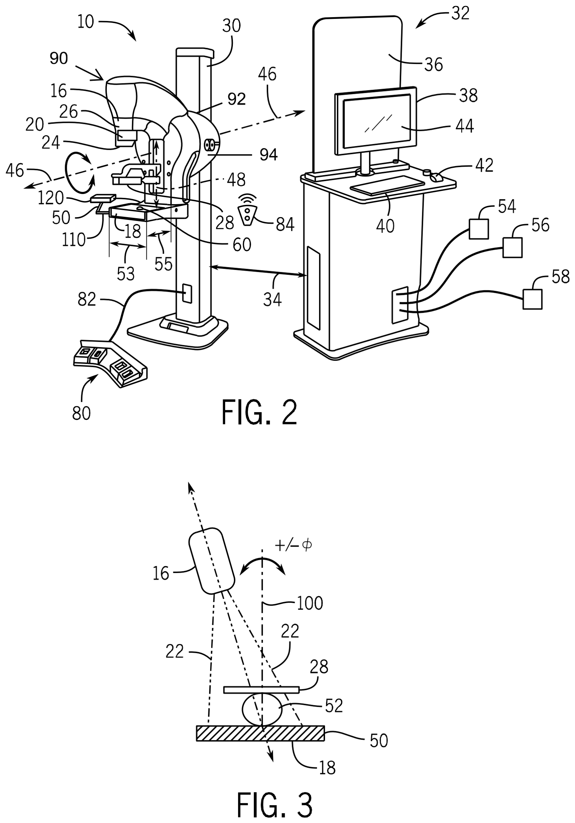

Referring now to FIGS. 2 and 3, the major components of an exemplary imaging system 10 formed as a mammography system 12 for imaging breast tissue according to an embodiment of the invention are shown. The system 10, such that disclosed in US Patent Application Publication No. US20200060632, entitled *Apparatus And Method For Mammographic Breast Compression,* the entirety of which is expressly incorporated herein by reference for all purposes, includes a radiation source/x-ray source 16, a radiation detector 18, and a collimator 20. The radiation source 16 is movable between a variety of imaging positions relative to the detector 18 and is operative to emit radiation rays 22 (FIG. 3) that are received by the radiation detector 18 to provide an image of an object, such as a breast 52. In embodiments, the system 10 may include a patient shield 24 mounted to the radiation source 16 via face shield rails 26 to prevent the patient's head from obstructing the radiation rays and protecting the patient from the radiation rays 22.

Referring still further to FIGS. 2 and 3, the system 10 also includes a motorized and/or manually adjustable compression paddle or plate 28 and a support structure 30 to which one or more of the radiation sources 16, radiation detector 18, and/or compression plate 28 are mounted. In embodiments, the system 10 includes a controller 32. The controller 32 may be a workstation having at least one processor/central processing unit/computer and a memory device/database that stores information and/or non-transitory instructions for the operation of various operational modes of the system 10 that are employed by the controller 32, as shown in FIG. 1 or, in other embodiments, the controller 32 may be embedded/integrated into one or more of the various components of the system 10 disclosed above. In embodiments, the controller 32 may be in electrical communication with the radiation source 16, radiation detector 18, and/or the compression plate 28 via a cable 34. As will be appreciated, in embodiments, the connection 34 may be a wireless connection. In embodiments, the controller 32 may include a radiation shield 36 that protects an operator of the system 10 from the radiation rays 22 emitted by the radiation source 16. The controller 32 may further include a display 38, a keyboard 40, mouse 42, and/or other appropriate user input devices that facilitate control of the system 10 via a user interface 44.

As further shown in FIGS. 2 and 3, the radiation source 16, along with the radiation detector 18, forms part of an x-ray system which provides x-ray imagery for the purpose of imaging a body part of a patient, such as breast 52. As stated above, the radiation source 16 emits the radiation rays 22 such that the radiation rays 22 travel from the radiation source 16 to the radiation detector 18. While the radiation rays 22 are discussed herein as being x-rays, it is to be understood that the radiation source 16 may emit other types of electromagnetic rays which can be used to image a patient. The radiation source 16 may be mounted to the support structure 30 such that the radiation source can rotate around an axis 46 in relation to the radiation detector 18, although movement of the radiation source 16 in paths other than rotation about a fixed axis, such as during digital breast tomosynthesis (DBT), are also envisioned. In embodiments, the radiation detector 18 may be configured to rotate or translate within its housing, such as in the directions indicated by arrows 53 and 55.

In the illustrated exemplary embodiment of FIG. 2 the radiation source 16 and the detector 18 are mounted to a gantry 90 that is secured to the support structure 30. The support structure 30 houses a translation mechanism 92 that is operably connected to the gantry 90. The translation mechanism 92 is operable to move the gantry 90 vertically with respect to the support structure 30 in order to position the gantry 90 at the appropriate height to accommodate the dimensions of the patient on which the system 10 is being utilized. The translation mechanism 92 is also operable to rotate the gantry 90 relative to the support structure 30 about the horizontal axis 46 in order to position the gantry 90 rotationally with regard to the patient, as necessary.

The gantry 90 includes a generally C-shaped body 94 with the radiation source 16 at one end and the detector 18 at the opposite end. In this configuration, regardless of the vertical and/or rotational orientation of the gantry 90, such as to position the radiation source 16 and detector 18 relative to the patient breast 52 to obtain x-ray images at various orientations, such as for craniocaudal (CC) or mediolateral oblique (MLO) views, among others, the radiation source 16 is disposed in alignment with the detector 18. In this position, the detector 18 is capable of receiving the x-rays 22 emitted from the radiation source 16 that pass through the portion of the patient, i.e., patient breast 52, located between the radiation source 16 and the detector 18 in order to generate image data for transmission to the control system 32 of the mammography device/system 10 to create/reconstruct a 3D image dataset for viewing by a physician, such as by using DBT, among other known methods.

Additionally, in another embodiment the radiation source 16 can be attached to the gantry 90 to rotate and/or move independently of the gantry 90 and detector 18 in order to enable the radiation source 16 to take a number of x-ray projections/images of the patient breast at various positions within a limited angular range relative to the detector 18, e.g., at angles between +/- 7.5°, +/- 12.5°, +/- 15°, +/- 25° and +/-60° relative to the object and/or vertical axis 100. The images obtained at the number of positions between these angles for the radiation source 16 can be used either for creation of stereoscopic images in a biopsy procedure using the system 10 or for DBT when operating the system 10 in an imaging mode.

As stated above, the radiation detector 18 receives the radiation rays 22 emitted by the radiation source 16. In embodiments, data regarding the radiation rays 22 received by the radiation detector 18 may be electrically communicated to the controller 32 from the radiation detector 18 via cable/electronic connection 34 such that the controller 32 generates one or more images which may be shown on the display 38 and stored in the memory device.

The compression plate 28 and motor (not shown) controlling the movement of the compression plate 28 is operative, in response to instruction from the controller 32 or in response to instructions from controller(s) on or near the mammography system 10, such as remote control, 84, or switch controllers 80 connected by cable 82, to move towards and away from the radiation detector 18 as indicated by arrows/compression axis 48 such that the compression plate 28 flattens and holds a body part, e.g., breast 52, in place against the surface 50 of the radiation detector 18. In this respect, the radiation detector 18 and the surface 50 thereof is referred to herein as a "compression surface or support plate" that cooperates with the compression plate 28 to compress and clamp a breast of a patient therebetween.

In an embodiment, the system 10 may further, or alternatively, include a biopsy tool 120, illustrated in FIG. 3. In such an embodiment, the radiation source 16, along with the radiation detector 18, forms part of an x-ray system which provides x-ray imagery for the purpose of guiding the biopsy tool 120, e.g., needle, to a suspect site within a body part of a patient. As shown in FIG. 3, in embodiments, the biopsy tool 120, may be disposed on a biopsy positioner 110 mounted to the support structure 30 such that it also rotates about the axis 46, in a manner similar to the radiation source 16, and/or moves in a vertical and/or horizontal direction, in a manner similar to the compression plate 28.

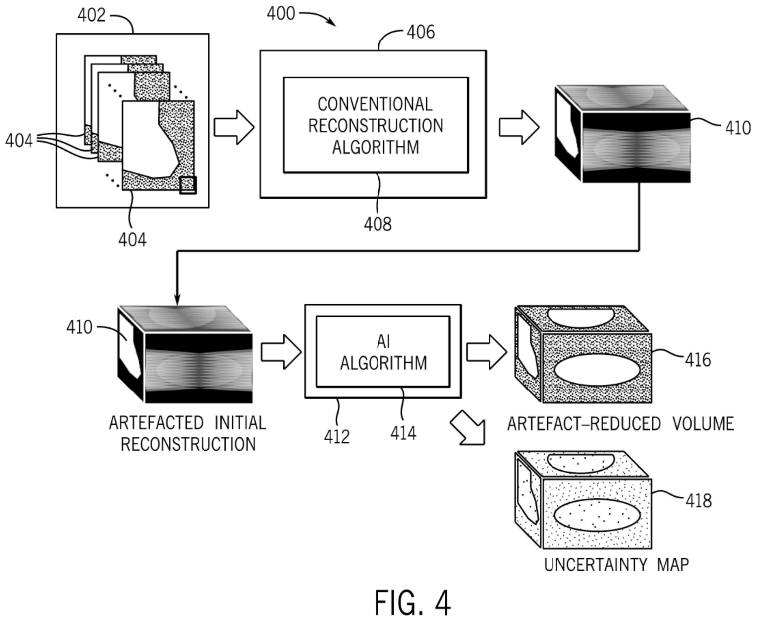

With reference now to FIG. 4, an exemplary embodiment of a method 400 of operation of the mammography system 10 is illustrated. In the method 400, in initial step 402 the radiation source 16 is operated to obtain a number of projection images 404 of the object/breast 52 at a number of different angular positions of the radiation source 16 relative to the breast 52 and detector 18, e.g., over a limited angular range, e.g., less than 180°, where an angular range of at least 180° is required for computed tomography (CT) imaging procedures, or less than +/- 60°, or less than +/-25°, or less than within +/-7.5°, each relative to the vertical axis 100 (FIG. 3), such as utilized in DBT imaging procedures. In step 406, these projection images 404 are utilized by a conventional reconstruction algorithm 408 which can be stored as a set of processor-executable and/or non-transitory instructions in memory within the system 10 accessible by the processor 32 to form a standard reconstruction volume 410.

In step 412, the standard reconstruction volume 410 is utilized by one or more separate artefact-reduction and/or resolution enhancement algorithms/artificial intelligences (Al)/neural networks (NN) 414 to form an artefact-reduced or quasi-isotropic volume 416. Examples of the one or more artefact-reduction and/or resolution enhancement algorithm/AI/NN 414 designed for DBT can be found in: D. Wu, K. Kim, and Q. Li, 'Digital Breast Tomosynthesis Reconstruction with Deep Neural Network for Improved Contrast and In-Depth Resolution', in 2020 IEEE 17th International Symposium on Biomedical Imaging (ISBI), Apr. 2020, pp. 656-659, A. Quillent et al., 'A Deep Learning Method Trained on Synthetic Data for Digital Breast Tomosynthesis Reconstruction', Medical Imaging with Deep Learning, 2023, and A. Quillent et al., 'Deep-Learning Uncertainty Estimation for Data-Consistent Breast Tomosynthesis Reconstruction'. In 21st International Symposium on Biomedical Imaging (ISBI 2024). Athens, Greece: IEEE Signal Processing Society and IEEE Engineering in Medicine and Biology Society, 2024, each of which are expressly incorporated herein by reference in their entirety for all purposes. The resolution enhancement algorithm/AI/NN 414 reduces the artefacts contained in the standard reconstruction volume 410 and in particular those artefacts illustrated within images along planes that are not parallel to the plane of the detector 18, (e.g., planes orthogonal to the detector 18, such as the coronal and sagittal planes) greatly improving the image quality of those images and enabling them to be effectively read for diagnostic and other purposes. In addition to the artefact-reduced volume 416, the resolution enhancement algorithm/AI/NN 414 can output an uncertainty map 418 graphically illustrating the assessment of the quality of the artefact-reduced or quasi-isotropic volume 416 and any images generated therefrom. Areas where the resolution enhancement algorithm/AI/NN 414 underperformed display high pixel intensities in the uncertainty map 418.

In an alternative embodiment, the processing of the projection images 404 by a first reconstruction algorithm 408 is omitted and the resolution enhancement algorithm/AI/NN 414 can be applied directly to projections 404, i.e., typically in the attenuation domain and produce an artefact reduced volume 416. Example of artefact-reduction and/or resolution enhancement algorithm/AI/NN 414 directly applied to projection images 404 with omission of the first reconstruction algorithm stage 408 can be found in: J. Teuwen et al., 'Deep learning reconstruction of digital breast tomosynthesis images for accurate breast density and patient-specific radiation dose estimation', Medical Image Analysis, vol. 71, p. 102061, Jul. 2021, which is expressly incorporated herein by reference in their entirety for all purposes.

With regard to the steps performed in the method 400, and the various post-processing steps to be described, these steps and the operation of each of the reconstruction algorithm 410 and the resolution enhancement algorithm/AI/NN 414 can be performed by the processor 32 utilizing processor-executable and/or non-transitory instructions stored in the memory device and accessible by the processor 32 regarding the operation of each of the mammography system 10 and the reconstruction algorithm 410 and the resolution enhancement algorithm/AI/NN 414.

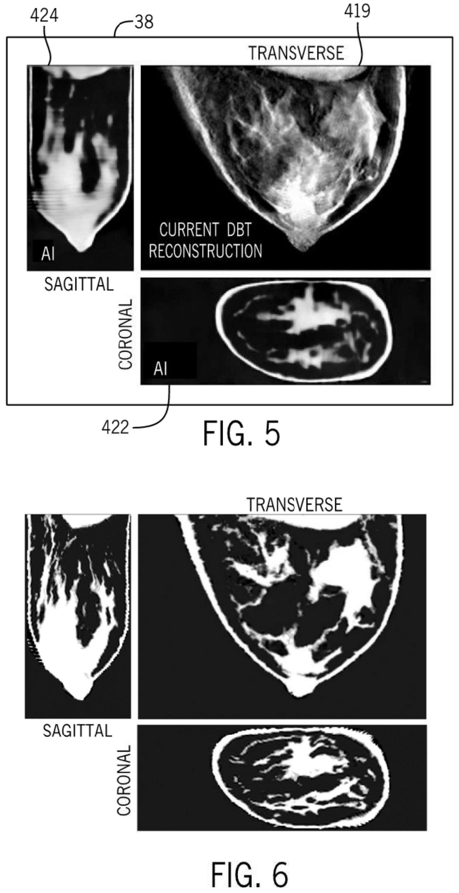

Looking now at FIGS. 5-15, once the artefact-reduced volume 416 has been produced as an output from the resolution enhancement algorithm/AI/NN 414 in step 412, different post-processing steps can be performed using the artefact-reduced volume 416.

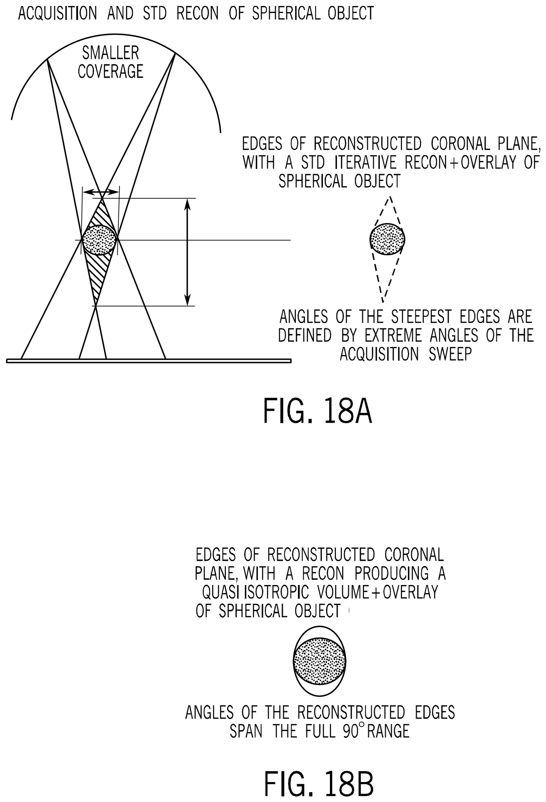

With particular reference to FIGS. 5-6, with the artefact-reduced volume 416, it is possible to produce views or images of the breast 52 with quasi-isotropic resolution along previously unused directions of sight or planes within the artefact-reduced and/or quasi-isotropic volume 416, such as along planes not parallel to the detector 18, along planes orthogonal to the detector 18, or along any line between the detector 18 and the focal point of the radiation source 16. In FIG. 5, a representation of the display 38 of the mammography system 10 is illustrated in which one or more standard reconstruction images, such as a transverse plane image 419 of the breast 52 are formed from the standard reconstruction volume 410 is shown. In addition, a coronal plane image 422 and a sagittal plane image 424 formed from the artefact-reduced and/or quasi-isotropic volume 416 are presented in conjunction with the transverse plane image 419. In some embodiments, the additional image planes, such as the coronal plane image 422 and the sagittal plane image 424, each have at least a quasi-isotropic resolution similar to that for the transverse plane image 419 obtained from the standard reconstruction volume 410, such that each provides useful diagnostic information regarding the imaged breast 52, in contrast to the images along these same planes produced from only the standard reconstructed volume 410, as shown in FIG. 1. With regard to the artefact-reduced and/or quasi-isotropic volume 416 and the enhanced quasi-isotropic resolution provided within the various plane images, e.g., the transverse plane image(s) 420, coronal plane image(s) 422 and/or sagittal plane image(s) 424, produced therefrom, the operation of the resolution enhancement algorithm/AI/NN 414 on the standard reconstruction volume 410 provides the artefact-reduced and/or quasi-isotropic volume 416 that contains edge information over the full 180° inside the breast 52 for typical breast textures, or close to it, as schematically illustrated in FIGS. 18A-18B and described in Quinto, E.T. Artifacts and Visible Singularities in Limited Data X-Ray Tomography. Sens Imaging, 9 (2017), the entirety of which is expressly incorporated herein by reference for all purposes.

In the current state of the art, the resolution in plane of regular DBT is typically a few tens of mm or better, i.e. the point spread function in-plane is typically 250 mm wide (FWHM - full width at half maximum). In addition, current resolution of regular DBT in the transverse direction is highly dependent on the dimension of the object in the plane direction. As a result, the artefact spread function associated with that plane can be computed from object size and sweep angle, with resulting resolution being centimetric or worse. (See Dalmonte & al. DOI: 10.1016/j.ejmp.2024.103300., which is incorporated herein by reference in its entirety for all purposes). The resolution enhancement algorithm/AI/NN 414 provides the quasi-isotropic improvement of the resolution in image planes 422,424 of the quasi-isotropic volume 416 that are orthogonal to the detector 18, are not parallel to the detector 18 and/or are oriented along any line between the detector 18 and the focal point of the radiation source 16, e.g., the coronal plane and the sagittal plane, from centimetric to millimetric in the z-direction.

In addition, FIG. 6 presents transverse, coronal and sagittal simulated images of the breast 52, compressed and positioned as in a standard DBT system, as would be obtained from a volume produced in a computed tomography (CT) imaging procedure that does not create the same artefacts in planes that are not parallel to the detector 18. As shown, the coronal plane image 422 and the sagittal plane image 424 obtained from the artefact-reduced and/or quasi-isotropic volume 416 closely approximate the isotropic resolution of images along the same planes obtained from the CT imaging procedure (FIG. 6), verifying the accuracy of the image data in the artefact-reduced and/or quasi-isotropic volume 416 and the various images, e.g., the coronal plane image 422 and the sagittal plane image 424, or any other image along a plane not parallel to the detector 18, produced therefrom.

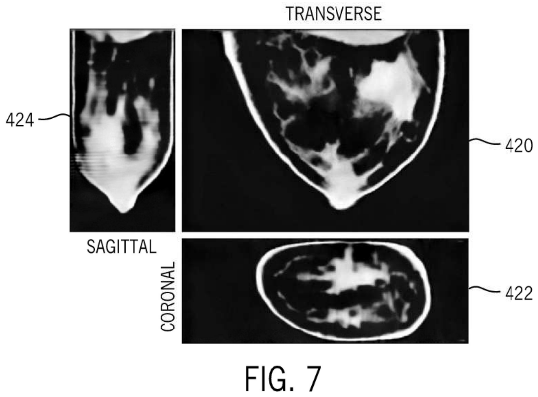

In one alternative embodiment shown in FIG. 7, the transverse plane image 420 presented on the display 38 can also be generated from the artefact-reduced and/or quasi-isotropic volume 416. Further, as an alternative to and/or in addition to one or more of the transverse plane image 420, the coronal plane image 422 and the sagittal plane image 424, the system 10 and/or processor 32 can reproject one or more images along any unacquired angle within the artefact-reduced and/or quasi-isotropic volume 416 with some specific post-processing, such as, but not limited to, the generation of a synthetic 2D image in any direction within the artefact-reduced and/or quasi-isotropic volume 416, including along each of the coronal/transverse/sagittal planes and others depending on the application, and not limited to images along the planes defined by the angles of the radiation source 16 at which the projection images 404 were acquired.

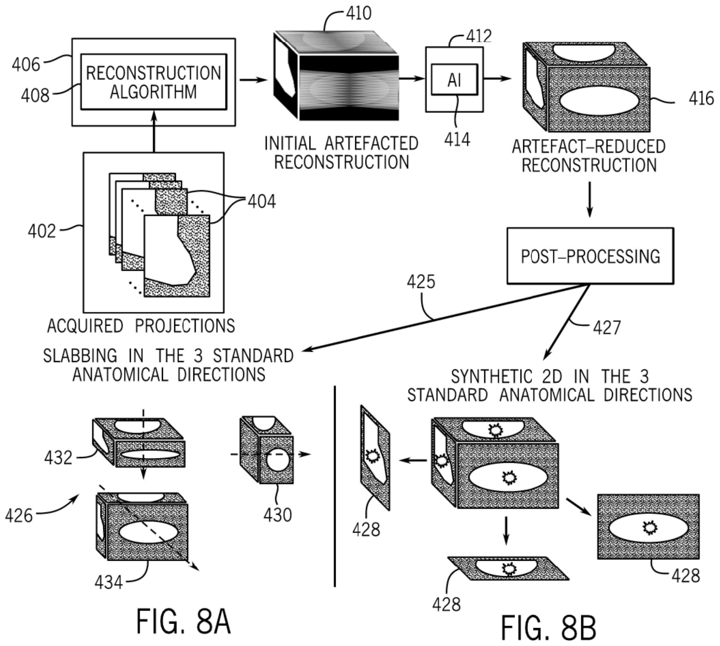

Referring now to FIG. 8A, as an alternative to the coronal plane image 422 and the sagittal plane image 424 shown in FIG. 5 and/or the transverse plane image 420 in FIG. 7, in order to reduce the amount of data to read for the users or to transfer in step 425 the processor 32 can operate to form the artefact-reduced and/or quasi-isotropic volume 416 into groups of planes including similar image data, or slabs 426. The slabs 426 can be oriented along any of the transverse, coronal and/or sagittal planes as a result in the enhanced image quality of the artefact-reduced and/or quasi-isotropic volume 416. The thickness of the slabs 426 across the artefact-reduced and/or quasi-isotropic volume 416 can be determined by the processor 32 based on an analysis of the similarity of the data across the planes of the artefact-reduced and/or quasi-isotropic volume 416, or on manually selected thickness for the slabs 426.

Alternatively, as shown in the exemplary embodiment of FIG. 8B, in step 427 the processor 32 can operated to produce a synthetic 2D image 428 along each of the of the transverse, coronal and/or sagittal planes, and/or any other desired planes not parallel to the detector 18 as a result in the enhanced image quality of the artefact-reduced and/or quasi-isotropic volume 416. The synthetic 2D image 428 effectively summarizes the information across the entire artefact-reduced volume 416 in the selected plane (transverse, coronal, sagittal) in a single 2D image to provide a quickly reviewable and readily assessable image for all image data within the artefact-reduced and/or quasi-isotropic volume 416.

In addition, in FIG. 7 the display 38 is illustrated as presenting a number of image(s) 420,422,424 along the transverse, coronal and sagittal planes, as well as other desired planes not parallel to the detector 18, as discussed above, at least some of which were obtained from the artefact-reduced and/or quasi-isotropic volume 416. As an alternative to the images 420,422,424, the display 38 can present images generated using different slabbing algorithm along different directions or planes. For example, the artefact-reduced volume 416 can be presented as a single 3D-slabbed volume shown from different directions, e.g., the transverse, coronal and sagittal planes. Alternatively, the display 38 can present a number of 2D-slabbed volumes 430,432,434 (e.g., 1 for each of the transverse, coronal and sagittal planes) synced on the display 38. Further, similarly to the images 420,422,424 in FIG. 5, the transverse plane slabbed volume 426 can be formed from the standard reconstruction volume 410, while the coronal plane slabbed volume 428 and the sagittal plane slabbed volume 430 can be formed from the artefact-reduced volume 416. The thickness of the slabs 430,432,434 produced in different orientations, for instance transverse and coronal, can differ.

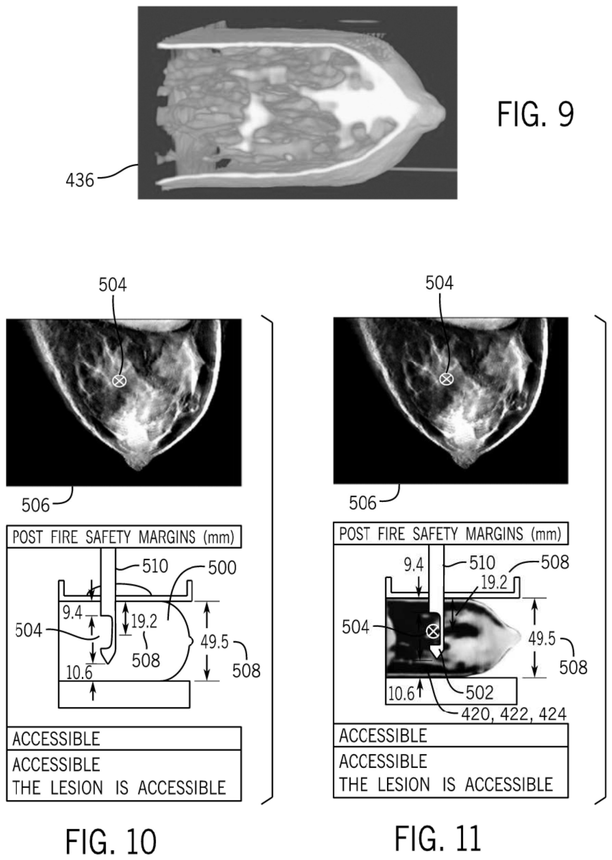

With reference now to FIG. 9, in addition to or separately from the above-described images 420-424 and slabbed volumes 430-434, an enhanced volume rendering 436 can also be formed from the artefact-reduced and/or quasi-isotropic volume 416. The enhanced volume rendering 436 is a representation of the artefact-reduced and/or quasi-isotropic volume 416 that enables a review of the entire exterior and interior structure of the imaged breast 52 at various rotational and cross-sectional positions of the enhanced volume 432.

Looking now at FIG. 10, in certain configurations the mammography system 10 includes a biopsy positioner 110 (FIG. 2) and biopsy device 120 (FIG. 2) positioned thereon and operable by the user via the control system 32 to perform a biopsy procedure. In prior art systems, as shown in FIG. 10A, during a biopsy procedure a simplified breast diagram 500, which is the same for all patients, is presented on the display 38 as a map to guide the movement of the needle 510 towards the biopsy target 504. In the performance of the biopsy procedure, the target 504 is selected on the in-plane images 506 (similar to 419) of the standard reconstruction volume 410. The expected needle trajectory 502 is also displayed on the diagram 500 along with the computed safety margins 508 for the trajectory 502. The trajectory 502 cannot be shown on the standard reconstructed planes 506,419 because the image quality for the images is too low. The needle 510 is then inserted into the breast 52 and the clinician can follow the progression of the needle 510 on the diagram 500.

Referring now to FIG. 11, the improved image quality of the artefact-reduced and/or quasi-isotropic volume 416 generated by the resolution enhancement algorithm/AI/NN 414 allows the substitution of either the artefact-reduced and/or quasi-isotropic volume 416,432 or an image 420,422,424 in place of the diagram 500. As a result, the display 38 presents a personalised map for the needle trajectory 502, target 504 and safety margins 508 for each patient. The use of the artefact-reduced and/or quasi-isotropic volume 416,432 or an image 420,422,424 as the map one the display 38 also enable the location of the needle 510 to be more precisely illustrated on the map and the size and location of the target 504 extent easier to visualize and determine.

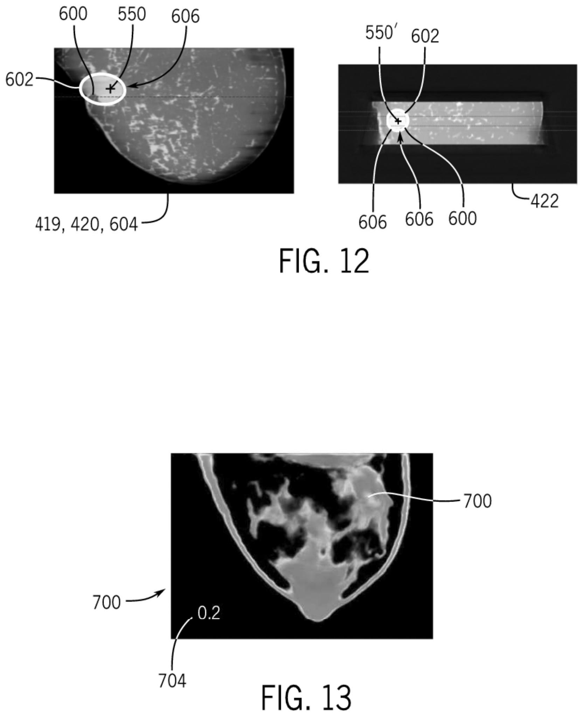

Looking now at FIG. 12, the output of the different steps 406 and 412 method 400 of FIG. 4 includes the standard reconstruction volume 410 and the artefact-reduced and/or quasi-isotropic volume 416. Since these respective volumes 410,416 are formed from the same projection images 404 of the breast 52 under a single compression, the volumes 410,416 can be registered to one another by the system 10 and/or processor 32 in any suitable known manner. As a result of this registration or synchronization between the standard reconstruction volume 410 and the artefact-reduced and/or quasi-isotropic volume 416, and/or quasi-isotropic images 420,422,424 a user can employ the user interface 44 to select a spot or area on the standard reconstruction volume 410, or standard reconstruction image/virtual image/projection created therefrom, presented on the display 38, and the processor 32 can present one or more orthogonal views, e.g., along the transverse, coronal and/or sagittal planes, or other virtual projections generated from the artefact-reduced and/or quasi-isotropic volume 416 at the same registered point in the artefact-reduced and/or quasi-isotropic volume 416.

Using the registration and/or synchronization between the standard reconstruction volume 410/standard reconstruction images 419 and the artefact-reduced and/or quasi-isotropic volume 416/quasi-isotropic images 420,422,424, when images 419,420,422,424 are presented on the display 38, such as in FIG. 5, movement by a user of a cursor 550 on one image 419,422,424 can be synchronized with movement of a separate cursor 550' disposed on one or more of the other images 419,422,424 illustrating a view along a different plane or axis to show the location of the cursor 550,550' in each of the displayed images 419,422,424.

In addition, due to the registration/synchronization between volumes 410 and 416, and associated reconstruction and quasi-isotropic images, the user interface 44 can be employed to manually annotate a three-dimensional region of interest (ROI) 600 in one image 419,420,422,424 or volume 426,428,430,432 and to indicate and compute volume and diameter of the same ROI 600 in all other virtual projections, images, volumes and/or axes, as opposed to the limited two-dimensional annotation on a single image or axis available on current mammography systems. As shown in FIG, 12, the annotation 602 of a single ROI 600 in transverse plane image 419,420,604 of the breast 52 can be translated into a volume 606 represented in a separately displayed coronal plane image 422,608 of the breast 52.

The annotation 602 can also be utilized by the processor 32 to perform an automatic or semi-automatic computation of the volume of the mass/lesion/ROI 600 identified within the annotation 602, which is currently unfeasible in DBT. This result can be stored and later accessed and/or employed to track any changes in the volume of the mass/lesion/ROI 600 over time, and to optionally propose a projection of the expected evolution based on the form and/or composition of the volume of the mass/lesion/ROI 600.

Referring now to FIG. 13, with the uncertainly map 418 generated along with the artefact-reduced and/or quasi-isotropic volume 416, it is possible to add an indication 700 of the computed uncertainty for a virtual projection or image 420,422,424 and/or volume 426,428,430 generated from the artefact-reduced and/or quasi-isotropic volume 416. In the exemplary embodiment illustrated in FIG. 13, the indication 700 can take the form of a colored overlay 702 located on the displayed image(s) 420,422,424 and/or volume(s) 426,428,430 in which different colors represent various levels of uncertainty within the individual pixels or voxels forming the image(s) 420,422,424 and/or volume(s) 426,428,430 generated from the artefact-reduced volume 416. In addition to the overlay 702 or as an alternative indication 700, the displayed image(s) 420,422,424 and/or volume(s) 426,428,430 can include a total uncertainty score 704 representing an overall calculated uncertainty for the image(s) 420,422,424 and/or volume(s) 426,428,430 generated from the artefact-reduced and/or quasi-isotropic volume 416. Further, in addition to the indication 700, should the uncertainty score for a displayed image 420,422,424 and/or volume 426,428,430 be above a predetermined threshold, which can be automatically or manually preset, the system 10 and/or processor 32 can present a warning regarding the high uncertainty value to the user on the display 38.

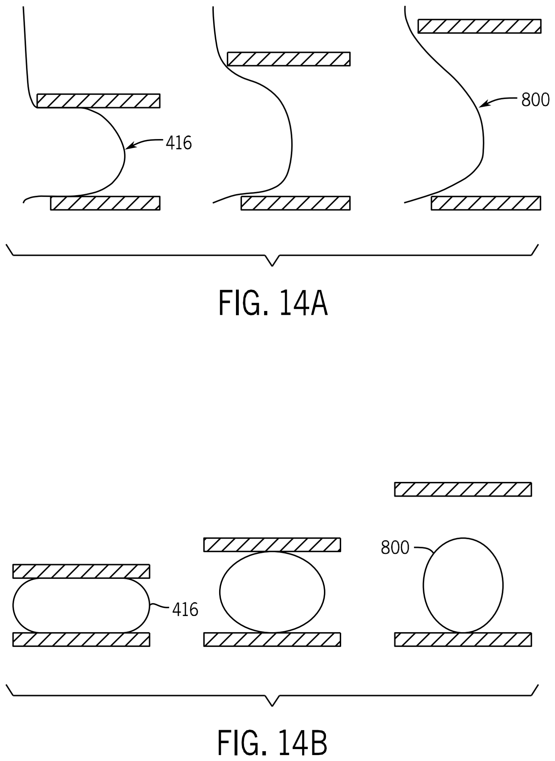

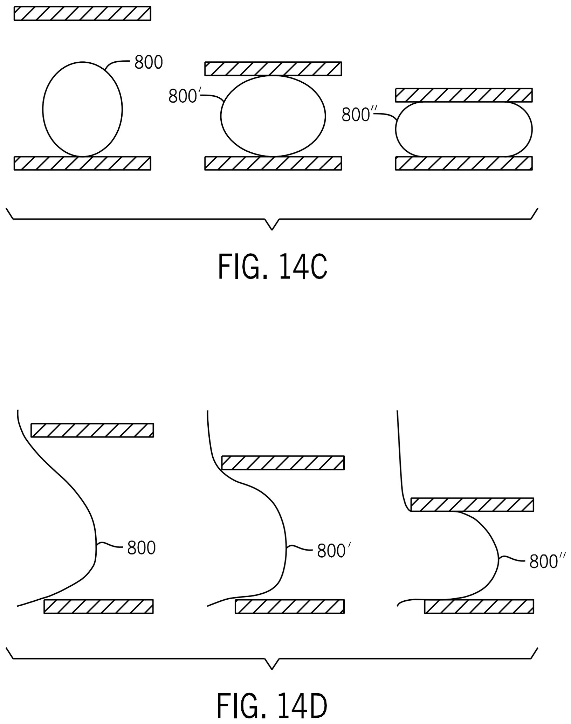

Looking now at FIGS. 14A-14D, after the output of the artefact-reduced and/or quasi-isotropic volume 416 from the method 400, it is possible to employ a conventional image segmentation on the artefact-reduced and/or quasi-isotropic volume 416 in order to assign materials (e.g., gland, fat, mass/cyst, etc.) to each voxel in the artefact-reduced and/or quasi-isotropic volume 416 using a standard image segmentation algorithm as utilized currently in magnetic resonance imaging (MRI) and CT imaging processes. Examples of these processes are disclosed in each of Frangi, A.F., Prince, J.L, and Sonka, M., (2024) Medical Image Analysis, Elsevier (DOI: https://doi.org/10.1016/C2015-0-06316-X), particularly with regard to convention methods in Part 3 and AI methods in Part 5, Chapter 18, and/or N. Geeraert et al 2014 Phys. Med. Biol. 59 4391; Comparison Of Volumetric Breast Density Estimations From Mammography And Thorax CT (DOI: 10.1088/0031-9155/59/15/4391), the entirety of which are each expressly incorporated herein by reference for all purposes.

With knowledge of the physical properties of each material type assigned to each voxel in the artefact-reduced and/or quasi-isotropic volume 416 via the image segmentation of the artefact-reduced and/or quasi-isotropic volume 416, and of the compression force applied to the breast 52 during the imaging procedure performed by the system 10 (as recorded by the system 10 via the compression paddle 28), the processor 32 can numerically invert the compression and gravity represented in the artefact-reduced and/or quasi-isotropic volume 416 to create a digital uncompressed breast volume 800, as schematically illustrated in FIGS. 14A-14B. The digital uncompressed volume 800 represents and can show the form of the breast 52 under only the influence of gravity, prior to the application of any compressive forces thereon.

As illustrated in FIG. 14C-14D, the digital uncompressed volume 800 can be digitally compressed to form a first modified digital volume 800' which is numerically modified/digitally compressed to show the form of the breast 52 under the effects of a simulated first compression force exerted on the breast 52 by the compression paddle 28 and the compression surface 50 of the detector 18. Additionally, in FIGS. 14C-14D, second modified digital volume 800" illustrates the further digital compression showing the effects of a simulated second compression force exerted on the breast 52 by the compression paddle 28 and the compression surface 50 of the detector 18.

As illustrated in FIGS. 14A-14D, the digital uncompressed breast volume 800 created from the artefact-reduced and/or quasi-isotropic volume 416 can be oriented into any simulated position and digitally compressed in any desired direction with a specified force in order to generate a synthetic image of the breast 52. The optimal compression direction, compression paddle position and breast position on the compression surface can be selected for the digital manipulation of the digital uncompressed breast volume 800 depending on the specific diagnostic needs. Thus, the digital uncompressed breast volume 800 can be used to create simulated volumes (800', 800"') from which can be obtained virtual or synthetic images of the breast 52, such as a synthetic cranial-caudal (CC) and/or mediolateral-oblique (MLO) image of the breast 52 for diagnostic purposes.

Also, with the system 10 and/or processor 32 able to generate multiple synthetic images of the breast 52, with each image created from the digital uncompressed breast volume 800 effectively registered to one another, when displaying multiple synthetic images of the breast 52 together, such as a synthetic CC image and a synthetic MLO image, with the user interface 44 a user could select an object and/or area within the CC image and the corresponding object or area could be shown in the MLO image in a much more accurate manner than is currently possible. Alternatively, separate methods 400 can be performed for the breast 52 in each of the CC and MLO orientations with a digital uncompressed breast volume 800 formed for each orientation. The resulting CC digital uncompressed breast volume and the MLO digital uncompressed breast volume can be registered to one another such that a selection of an object within one of the CC digital uncompressed breast volume or the MLO digital uncompressed breast volume would illustrate the same object in an image or plane of the other of the CC digital uncompressed breast volume and the MLO digital uncompressed breast volume.

Further, the synthetic images, e.g., the synthetic CC and/or MLO image(s) can be combined or utilized in conjunction with an actual image of the breast 52, e.g., an actual CC and/or MLO image(s), such as to enhance the actual image of the breast 52.

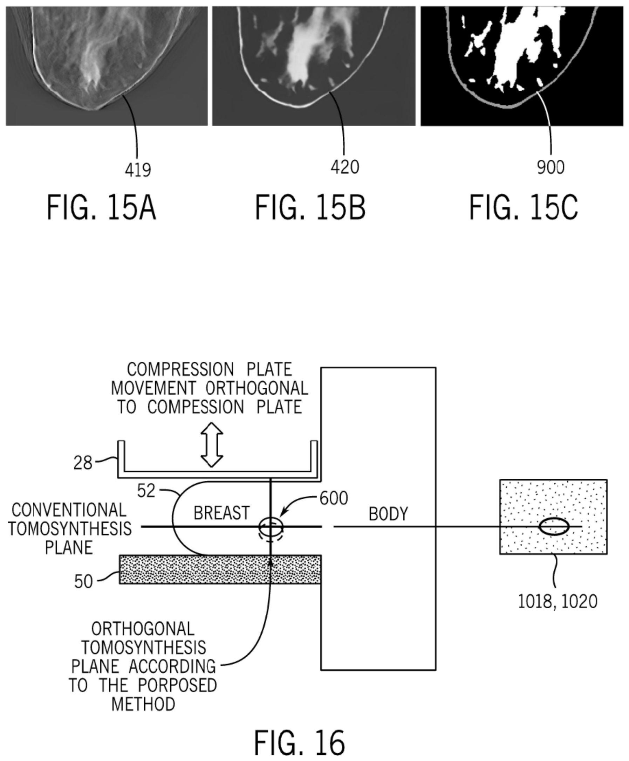

Looking now at FIGS. 15A-15C, the artefact-reduced and/or quasi-isotropic volume 416 can also be employed in an assessment of the tissues forming the breast 52. More specifically, in order to determine a density for the breast 52, as described previously with regard to FIGS. 14A-14D, the artefact-reduced and/or quasi-isotropic volume 416 can be processed using an image segmentation algorithm to assign materials to each voxel in the artefact-reduced volume 416. Using the results of the image segmentation, e.g., assigning a material or tissue type to a particular voxel of the artefact-reduced and/or quasi-isotropic volume 416 based on the intensity value of the voxel within the artefact-reduced and/or quasi-isotropic volume 416, a breast density can be calculated as a ratio of the number of voxels classified as gland and the number of voxels classified as fat.

In particular, in the exemplary illustrated embodiment of FIG. 15A, an image of the breast 52 obtained from the standard reconstruction volume 410 is shown for comparison with the image obtained from the artefact-reduced and/or quasi-isotropic volume 416 in FIG 15B, with FIGS. 15A-B presenting the same planar view of the breast 52. Using the image in FIG. 15B, the results of the image segmentation described previously, e.g., for analyzing the intensity of each pixel in the image of FIG. 15B, can be employed to create a material map/image 900 of FIG. 15C illustrating the types and locations of the various tissues presented in the image. With the illustrated categories of tissue assigned to the pixels in the image of FIG. 15C being either "gland" or "fat, a breast density can be calculated as a ratio of the number of pixels classified as gland and the number of pixels classified as fat. As a result, the map 900 formed by the system 10 and/or processor 32 provides an enhancement to the utility of the system 10 for quantitative imaging of a breast, where the system 10 and/or processor 32 can define and display new units such as a calibrated gland/fat ratio for the imaged breast 52, similar to Hounsfield units employed in CT imaging, in order to provide a better estimation of the gland density and location, resulting in a more precise calculation of the radiation dose and/or average glandular dose that is received by the patient.

Referring now to FIG. 16, with the ability of the artefact-reduced and/or quasi-isotropic volume 416 to provide images in planes that are not perpendicular to the detector 18, the system 10 and/or processor 32 can additionally perform certain mechanical imaging procedures, on the breast 52, including compression or strain x-ray elastography. As cancerous and noncancerous lesions, as well as other tissues of different types, such as other benign and malignant lesions, will demonstrate differing amounts of tissue motion relative to the normal surrounding breast tissue when minimal pressure is applied, strain x-ray elastography is a qualitative method that measures stiffness based on soft-tissue distortion and/or displacement caused by different levels of compression applied to the tissue.

As schematically illustrated in FIG. 16, in the operation of a mammography imaging system 10 to perform x-ray imaging of a breast 52 for screening or diagnostic purposes, the breast 52 is compressed on and against the detector 18 using a compression plate or paddle 28 with specified or selected compression force, such as via a motor operably connected to the compression plate 28 and controlled by the controller 32. The compression force applied by the compression plate 28 is monitored and recorded by the processor 32, such that that amount of compressive force applied to the breast 52 is closely controllable.

As stated previously, to provide useful diagnostic information on the imaged breast 52, the prior art images obtained of the breast 52 using the mammography imaging system 10 are required to be either projection images 404 oriented perpendicularly to the detector 18 and compression plate 28 (which have significantly low resolution, and thus are not useful for diagnostic purposes), or reconstructed planar images oriented parallel to the detector 18 and the compression plate 28 (with much higher resolution for diagnostic purposes). Consequently, the prior art reconstructed images along the transverse plane are "blind" to displacements in the direction of the compression force, e.g., changes in the height of any lesions within the breast 52. With the enhancement of the resolution of reconstructed images oriented along planes orthogonal to the detector 18 and compression plate 28, i.e. in the direction of the compression force and resulting displacements, provided by the method of FIG. 4, it is possible to adapt elastography to breast tomosynthesis.

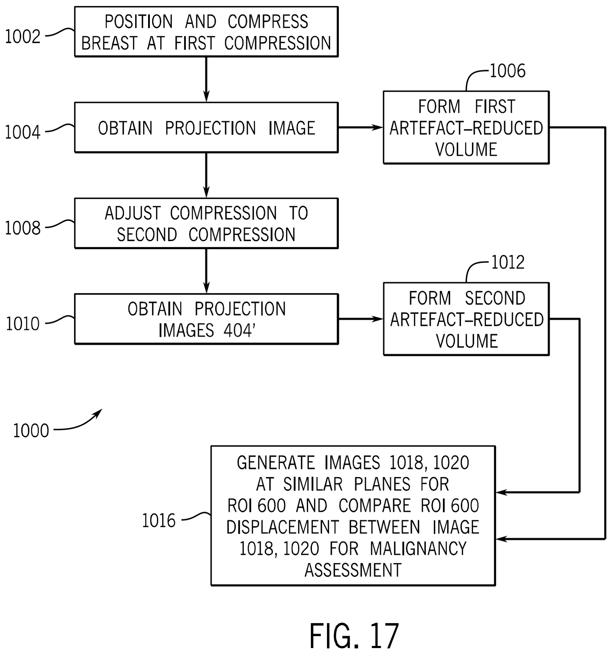

More specifically, referring to FIGS. 2, 4, 16 and 17, in the method 1000 initially in step 1002 the breast 52 is positioned on the compression surface 50 of the detector 18 and the compression paddle 28 is moved to compress the breast 52 under a usual or first compression force. In step 1004, a DBT imaging procedure is performed on the breast 52 to obtain a first set of projection images 404 of the breast 52 at various angular positions of the radiation source 16 relative to the detector 18. In step 1006, the first set of projection images 404 are employed in the method 400 of FIG. 4 to produce a first artefact-reduced and/or quasi-isotropic volume 416. In step 1008, the compression paddle 28 is moved vertically to produce a small change in the compression force (increased or decreased) on the breast 52, and/or horizontally towards or away from the gantry 90 to generate differential displacements of structures and/or lesions within the breast 52 due to the hysteresis of the tissue forming the lesions behaving in at least partially either an elastic mode or an inelastic mode depending on the type of tissue forming the lesions. In a subsequent step 1010, a second DBT imaging procedure is performed on the breast 52 to obtain a second set of projection images 404' of the breast 52 at various angular positions of the radiation source 16 relative to the detector 18. In step 1012, the second set of projection images 404' are employed in the method 400 of FIG. 4 to produce a second artefact-reduced and/or quasi-isotropic volume 416'.

In step 1014, images 1018,1020 of similar planes that are orthogonal to the plane of the detector 18 are obtained from each artefact-reduced and/or quasi-isotropic volume 416,416' and are jointly exploited or compared by the processor 32 and/or other outside computing device (not shown) to provide information or evidence differential local displacements of and/or mechanical properties of any lesions present in the images 1018,1020 to diagnostically determine the form of those lesions, using any one or more known methods such as subtraction, identification of similar structures, determining and showing as images the displacement vectors or their modules between homologous points, correlation images, etc. Some examples of the analytical processes to be employed can be any one or more of the following methods, each of which is expressly incorporated herein by reference in its entirety for all purposes: Ramião, N.G., Martins, P.S., Rynkevic, R. et al. Biomechanical properties of breast tissue, a state-of-the-art review. Biomech Model Mechanobiol 15, 1307-1323 (2016); Zhou, Cong, Hainsworth, Brent, Sydney, Maxwell, Lee, Michael, Ormsby, Zane, Haggers, Marcus and Chase, J. Geoffrey. "Structural health monitoring of tissue mechanics for non-invasive diagnosis of breast cancer" at - Automatisierungstechnik, vol. 66, no. 12, 2018, pp. 1037-1050; Nitta, N., Shiina, T. (2005). Breast Tissue Assessments Based on High Order Mechanical Properties. In: Ueno, E., Shiina, T., Kubota, M., Sawai, K. (eds) Research and Development in Breast Ultrasound. Springer, Tokyo.

With this information, the artefact-reduced and/or quasi-isotropic volumes 416,416' can be used assess the malignancy of any lesions within the imaged breast 52 based on the determination of the stiffness of the tissue forming the lesions from the images 1018,1020 of similar planes that are orthogonal to the plane of the detector 18 generated from the artefact-reduced and/or quasi-isotropic volumes 416,416'.

In addition, the artefact-reduced and/or quasi-isotropic volume 416 can be employed in conjunction with image(s) and/or volume(s) obtained of the breast 52 using other imaging modalities, including but not limited to MRI, bCT, and ultrasound (US), to enhance their diagnostic utility. For example, as discussed with regard to FIG. 12, an annotation made in a view, slab or image along one plane 420,422,424 can be translated into a view or image along another plane 420,422,424 due to the correspondence of geometry in the respective views/images 420,422,424 relative to the artefact-reduced volume 416 from which the views/images 420,422,424 were generated. Further, if a volume of the breast 52 were obtained using a different imaging modality, including but not limited to US or MRI, that volume can be registered to the artefact-reduced and/or quasi-isotropic volume 416 using known cross-modality registration processes. As a result, an annotation 602 made in the view(s)/image(s) 420,422,424 can be translated into the alternative-modality volume, or vice-versa. Thus, an annotation 602 made in one of the artefact-reduced and/or quasi-isotropic volume 416 or the alternative-modality volume can be employed by the system 10 and/or processor 32 to determine and present on the display 38 a selected view or image from the alternative-modality volume and images 420,422,424 along each plane, i.e., transverse, coronal, sagittal, that intersect at the annotation 602 to allow for the precise location of a lesion or tumor across modalities.

In still another exemplary embodiment, the compression paddle 28 can be formed from a material transparent to ultrasound waves, such as polymethylpentene sold under the name TPX by Mitsui Chemicals of Japan. Initially, the breast 52 can be compressed using the compression paddle 28 to perform a DBT imaging procedure/acquisition on the compressed breast 52, as described herein. Subsequently, the artefact-reduced/quasi-isotropic DBT volume 416 can be generated from the projection images 404 obtained from the acquisition. With a compression paddle 28 made of this type of material, after compression of a breast 52 and the performance of DBT imaging procedure/acquisition on the compressed breast 52, an ultrasound imaging system/ultrasound probe (not shown) can be utilized to obtain ultrasound (U/S) images through the paddle while leaving the breast 52 under the same compression as during the DBT acquisition. With the artefact-reduced and/or quasi-isotropic volume 416, it is possible to identify the plane of the U/S images being obtained through the breast 52, e.g., the planes orthogonal to the compression paddle 28 on which the U/S probe is positioned, and to superimpose the U/S image on or over the quasi-isotropic volume 416 after performing calibration between the artefact-reduced and/or quasi-isotropic volume 416 and the U/S images and/or system by using depth, by manually making identified findings in U/S images and the image plane of the artefact-reduced and/or quasi-isotropic volume 416 coincide, and/or by using known properties of the U/S system, e.g., probe type, U/S frequency, and properties of the breast tissue, among others.

In addition, with this configuration for the imaging system 10 and U/S transparent compression paddle 28 it is possible to obtain the projection images 404 of the breast 52 under compression to generate the artefact-reduced and/or quasi-isotropic volume 416, as described previously. A user and/or computer system, such as the processor 32, can subsequently identify a suspicious finding in a location of the breast 52, e.g., either within the regular or standard image planes or within planes obtained from the quasi-isotropic volume 416 according to the methods described previously.

If a suspicious finding is located, the breast 52 can be further analyzed by obtaining U/S images of the breast 52 in a plane benefitting from the invention, i.e. sensibly perpendicular to the paddle 28, where the U/S image can be acquired under the same compression or using a similar compression after repositioning the breast 52 in a sensibly similar position. After obtaining the U/S image, the U/S image can be registered with the quasi-isotropic/artefact-reduced volume 416, and/or any DBT plane image formed therefrom, according to methods of the disclosure discussed previously in order to enhance the diagnostic capabilities of the imaging system 10 in a manner distinct and improved from the simple re-registration of images obtained from different imaging modalities.

In addition, with respect to the generation of the digital uncompressed breast volume 800 from the artefact-reduced volume 416, as discussed previously with regard to FIGS. 14A-14D, the digital uncompressed breast volume 800 or the artefact-reduced volume 416 can be visually compared with image(s) and or volume(s) obtained of the breast 52 using other imaging modalities.

For example, a digitally compressed volume of the breast 52 obtained from an US or MRI imaging procedure can be compared with the artefact-reduced volume 416 of the breast 52 obtained via the method of FIG. 4.

Further, an uncompressed volume of the breast 52 obtained from an US or MRI imaging procedure can be compared with the digitally uncompressed volume 800 produced from the artefact-reduced volume 416.

Also, the digitally uncompressed volume 800 can be used directly or modified to approximate any similar compressed, decompressed or uncompressed form/volume of the breast 52 obtained using a different imaging modality for the purposes of registering the respective volumes with one another.

In addition, the artefact-reduced volume 416 can be used to define a coordinate system for the artefact-reduced volume 416. This coordinate system can be employed within any image(s) 420,422,424 created from the artefact-reduced volume 416 and applied through the registration of the artefact-reduced volume 416 with volumes of the breast 52 from other modalities to allow a precise cross-modality determination of the location a lesion, tumor or mass between he registered volumes.

Still further, the digital uncompressed breast volume 800 generated from the artefact-reduced volume 416 can be manipulated to simulate and/or provide synthetic image(s) and/or volume(s) of the breast 52 that would be obtained utilizing other imaging modalities.

In still another exemplary embodiment, the process in the method 400 for obtaining the projections 404 can be performed as a contrast-enhanced DBT or other contrast-enhanced imaging procedure, thereby enhancing the ability of the processor 32 to distinguish the locations of different tissue types or materials within the imaged breast 52 as a result of the dye, e.g., an iodine-based dye, injected into the breast 52 prior to performing the contrast-enhanced imaging procedure.

Finally, it is also to be understood that the imaging system 10 or any separate computing device employed to perform any of the methods 400,1000, etc. and/or processes described herein may include the necessary electronics, software, memory, storage, databases, firmware, logic/state machines, microprocessors, communication links, displays or other visual or audio user interfaces, printing devices, and any other input/output interfaces to perform the functions described herein and/or to achieve the results described herein. For example, as previously mentioned, the system 10 and/or separate computing device may include at least one processor and system memory/data storage structures, which may include random access memory (RAM) and non-transitory, read-only memory (ROM). The at least one processor of the system 10 and/or separate computing device may include one or more conventional microprocessors and one or more supplementary co-processors such as math co-processors or the like. The data storage structures of the system 10 and/or separate computing device discussed herein may include an appropriate combination of magnetic, optical and/or semiconductor memory, and may include, for example, RAM, ROM, flash drive, an optical disc such as a compact disc and/or a hard disk or drive.

Additionally, a software application that adapts the controller to perform the methods disclosed herein may be read into a main memory of the at least one processor from a computer-readable medium. The term "computer-readable medium", as used herein, refers to any medium that provides or participates in providing instructions to the at least one processor of the system 10 (or any other processor of a separate computing device employed to perform the methods and/or processes described herein) for execution. Such a medium may take many forms, including but not limited to, non-volatile media and volatile media. Non-volatile media include, for example, optical, magnetic, or optomagnetic disks, such as memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, a RAM, a PROM, an EPROM or EEPROM (electronically erasable programmable read-only memory), a FLASH-EEPROM, any other memory chip or cartridge, or any other medium from which a computer can read.

While in embodiments, the execution of sequences of instructions in the software application causes at least one processor to perform the methods/processes described herein, hard-wired circuitry may be used in place of, or in combination with, software instructions for implementation of the methods/processes of the present invention. Therefore, embodiments of the present invention are not limited to any specific combination of hardware and/or software.

It is understood that the aforementioned compositions, apparatuses and methods of this disclosure are not limited to the particular embodiments and methodology, as these may vary. It is also understood that the terminology used herein is for the purpose of describing particular exemplary embodiments only, and is not intended to limit the scope of the present disclosure which will be limited only by the appended claims.

## Claims

1. A method for providing quasi-isotropic images (420,422,424) of an object (52), the method comprising the steps of:
a. providing one or more projection images (404) obtained of the object (52) within an angular range of less than 180 degrees relative to the object (52);
b. reconstructing a quasi-isotropic volume (416) from the one or more projection images (404) with an artefact-reduction and resolution enhancement artificial intelligence (414); and
c. generating one or more quasi-isotropic images (420,422,424) from the quasi-isotropic volume (416).

2. The method of claim 1, wherein the one or more quasi-isotropic images (420,422,424) comprises an image plane along at least one plane orthogonal to the detector (18) or containing any line between a detector (18) and a focal point of a radiation source (16) of an imaging system (10) utilized for obtaining the one or more projection images (404).

3. The method of claim 2, wherein the one or more quasi-isotropic images (420,422,424) comprises at least one of a plane, a 2D image, a slab (420,432,434) or a 2D synthetic image (428), and combinations thereof.

4. The method of claim 2, wherein further comprising the steps of:
a. generating a standard reconstruction volume (410) from the one or more projection images (404);
b. generating one or more standard images (419) from the standard reconstruction volume (410);
c. registering the one or more quasi-isotropic images (420,422,424) to the standard reconstruction volume (410); and
d. synchronizing movement of a cursor (550) in each of the one or more quasi-isotropic images (420,422,424) and the one or more standard images (419).

5. The method of claim 3, further comprising the step of employing the one or more quasi-isotropic images in a biopsy procedure.

6. The method of claim 1, wherein the object (52) is a breast (52) and wherein the method further comprising the step of classifying a tissue type for individual voxels in the quasi-isotropic volume (416).

7. The method of claim 6, further comprising the step of numerically decompressing the quasi-isotropic volume (416) into a digital uncompressed volume (800).

8. The method of claim 7, further comprising the step of manipulating the digital uncompressed volume (800) to form additional views of the breast (52).

9. The method of claim 7, further comprising the steps of:
a. generating a standard reconstruction volume (410) from the one or more projection images (404);
b. registering the digital uncompressed volume (800) with the standard reconstructed volume (410).

10. The method of claim 1, further comprising the steps of:
a. generating an uncertainty score (704) for each voxel in the one or more quasi-isotropic images (420,422,424); and
b. displaying the uncertainty scores (704) along with the one or more quasi-isotropic images (420,422,424).

11. A mammography imaging system (10) comprising:
a. a radiation source (16) operable at to emit radiation,
b. a detector (18) alignable with the radiation source (16), the detector having a surface (50) on which a breast (52) to be imaged is adapted to be positioned;
c. a controller (32) operably connected to the radiation source (16) and the detector (18) to control the operation of the radiation source (16) and detector (18) to generate image data of the breast (52) in an imaging procedure performed by the imaging system (10), the controller (32) including a central processing unit and interconnected database containing processor-executable instructions for processing the image data from the detector to create one or more projection images (404) of the breast (52),
d. a display (38) operably connected to the controller (32) for presenting information to a user; and
e. a user interface (44) operably connected to the controller (32) to enable user input to the controller (32),
wherein the controller (32) is configured to apply a resolution enhancement artificial intelligence (414) to the one or more projection images (404) to reconstruct a quasi-isotropic volume (416) of the breast (52), and to generate one more one or more quasi-isotropic images (420,422,424) from the quasi-isotropic volume (416).

12. The imaging system of claim 11, wherein the imaging system (10) is a mammography imaging system (12) including a compression paddle (28) moveable relative to the detector surface (50) by the controller (32) to compress a breast (52) therebetween, and wherein the controller (32) is configured to determine a property of tissue forming a lesion (600) in the breast (52) from a comparison of one or more first quasi-isotropic images (420,422,424) obtained at a first compression with one or more second quasi-isotropic images (420,422,424) obtained at a second compression to enable differentiation of tissues forming the lesions (600) from other breast tissue as a result of determining mechanical properties of the tissue within the imaged breast (52).

13. The imaging system of claim 11, wherein the controller (32) is further configured to classify a tissue type for individual voxels in the quasi-isotropic volume (416).

14. The imaging system of claim 13, wherein the controller (32) is further configured to numerically decompress the quasi-isotropic volume (416) into a digital uncompressed volume (800).

15. The imaging system of claim 13, wherein the controller (32) is further configured to reconstruct a standard reconstruction volume (410) from the one or more projection images (404), to generate one or more standard reconstruction images (419) from the standard reconstruction volume (410), to register the one or more quasi-isotropic images (420,422,424) to the one or more standard reconstruction images (419) and to synchronize movement of a cursor (500) in each of the one or more quasi-isotropic images (420,422,424) and the one or more standard reconstruction images (419).
